# EUROPEAN PATENT SPECIFICATION

(11) **EP 1 587 792 B1**
(45) Date of publication and mention of the grant of the patent: **27.08.2014**
(21) Application number: 04705547.0
(22) Date of filing: 27.01.2004
(51) Int. Cl.: A61K 31/4422, A61P 9/10, A61P 9/12

(54) **STABLE AMORPHOUS AMLODIPINE CAMSYLATE, PROCESS FOR PREPARING SAME AND COMPOSITION FOR ORAL ADMINISTRATION THEREOF**
STABILES AMORPHES AMLODIPINCAMSYLAT,VERFAHREN ZU DESSEN HERSTELLUNG UND ZUSAMMENSETZUNG ZU DESSEN ORALERVERABREICHUNG
AMLODIPINE CAMSYLATE STABLE ET AMORPHE, PROCEDE DE PREPARATION ASSOCIE ET COMPOSITION POUR L'ADMINISTRATION ORALE

(30) Priority: 27.01.2003 KR 2003005227
(43) Date of publication of application: 26.10.2005
(73) Proprietor: Hanmi Science Co., Ltd., Gyeonggi-do 445-813 (KR)
(72) Inventor: MOON, Young Ho Hwanggolmaeul Jugong Apt., Suwon-si, Kyungki-do 442-470 (KR); KIM, Nam Du, Osan-si, Kyungki-do 447-290 (KR); LEE, Kyung Ik, Incheon 405-231 (KR); LEE, Gwan Sun, Seoul 138-160 (KR); WOO, Jong Soo, Suwon-si, Kyungki-do 440-300 (KR); CHANG, Young Kil, 138-180 Seoul (KR)
(74) Representative: Goddar, Heinz J.
(86) International application number: PCT/KR2004/000136
(87) International publication number: WO 2004/067512

(56) References cited:
- WO-A1-02/053135
- WO-A1-02/053538
- WO-A1-02/053539
- WO-A1-02/079158

## Description

### Field of the Invention

The present invention relates to an amorphous form of amlodipine camsylate which has a high solubility and good storage stability, and a composition for oral administration comprising same.

### Background of the Invention

Amorodipine, a generic name for the compound (3-ethyl-5-methyl-2-(2-aminoethoxy-methyl)-4-(2-chlorophenyl)-6-methyl-1,4 -dihydro-3,5-pyridine dicarboxylate), is a long-term calcium-channel blocker useful for treating cardiovascular disease such as angina pectoris, hypertension and congestive cardioplegia.

Amlodipine in the form of a free base is useful for pharmaceutical use, but it shows low stability. Therefore, it is preferably administrated in the form of a salt of a pharmaceutically acceptable acid.

Korean Patent Publication No. 1995-6710 suggests that a pharmaceutically acceptable salt must meet four physicochemical requirements: high solubility, good stability, non-hygroscopicity and processibility for tablet formulation.

Korean Patent Publication No. 1995-7228 discloses that amlodipine benzenesulfonate (hereinafter, "amlodipine besylate") shows suitable properties for pharmaceutical formulation including high solubility and good stability.

The present inventors have also suggested in WO 02/079158 A1 that a crystalline amlodipine camsylate is pharmaceutically stable.

### Summary of the Invention

It is a primary object of the present invention to provide an improved form of amlodipine camsylate which has higher stability and solubility than conventional amlodipine salts.

In accordance with another aspect of the present invention, there is provided amorphous amlodipine camsylate prepared by dissolving crystalline amiodipine camsylate in an organic solvent and removing the solvent from the resulting solution according to claim 1.

In accordance with still another aspect of the present invention, there is provided a pharmaceutical composition for treating cardiovascular disease comprising the amorphous amlodipine camsylate as an active ingredient according to claim 2.

### Brief Description of the Drawings

The above and other objects and features of the present invention will become apparent from the following description of the invention taken in conjunction with the following accompanying drawings, which respectively show:
FIG. 1: an X-ray diffraction scan of crystalline amlodipine camsylate;
FIG. 2: an X-ray diffraction scan of the inventive amorphous amlodipine camsylate;
FIG. 3: a differential scanning calorimetry (DSC) scan of crystalline amlodipine camsylate; and
FIG. 4: a differential scanning calorimetry (DSC) scan of the inventive amorphous amlodipine camsylate.

### Detailed Description of the Invention

The inventive amlodipine camsylate is substantially amorphous and thermodynamically stable, and has a high water solubility and good storage stability.

The inventive amorphous amlodipine camsylate; can be prepared by dissolving a crystalline form of amlodipine camsylate in an organic solvent and removing the solvent from the resulting solution to obtain an amorphous form of amlodipine camsylate.

Examples of the organic solvent which may be suitably used in the present invention includes acetone, methanol, acetonitrile, tetrahydrofurane and dioxane, methyl or ethyl acetate, methylene chloride, chloroform and a mixture thereof, preferably a mixture of ethanol and methylene chloride.

The used solvent can be removed by rapid solvent evaporation, spray drying, roller drying, solvent precipitation or freeze drying, among which spray drying is preferred since the resulting amorphous amlodipine camsylate is thermodynamically stable. More preferably, a closed cycle spray drying system which can recycle the drying medium may be used in terms of safety and economical efficiency of the process.

In spray drying, an inert gas such as argon, nitrogen and carbon dioxide or air may be used as a drying gas. Inflow and outflow temperatures thereof may be dependent on the boiling point of the solvent used. For- example, the inflow temperature may range 50 to 140 °C, preferably 60 to 125 °C and the outflow temperature may range 45 to 100 °C, preferably 50 to 80 °C. By employing appropriate spray drying conditions, a product having a uniform particle size may be formed.

In spray or roller drying, a solvent having a boiling point (b.p.) lower than the coagulation point of the inventive product is preferably used. When the drying is carried out under an atmospheric pressure, a solvent having b.p. of 80°C or less may be preferably used. If the drying is carried out under a reduced pressure, a solvent having b.p. higher than 80 °C may be used.

In solvent precipitation, crystalline amlodipine camsylate may be dissolved in a suitable organic solvent followed by adding a nonpolar solvent in a suitable amount thereto to induce precipitation of the inventive product. A preferred nonpolar solvent used in the precipitation include ethers such as isopropyl ether or aromatic hydrocarbons such as benzene and toluene. The nonpolar solvent should be at least partially miscible with the suitable organic solvent. A representative example of solvent combination is dichloromethane/methanol/isopropylether. The precipitated solid is preferably subjected to rapid filtration and drying, to avoid the formation of crystals. Also, a carrier gas such as air may be used to generate bubbles in solution.

Also, the solvent precipitation may be directly applied to the reaction mixture obtained after the formation of crystalline amlodipine camsylate.

Meanwhile, freeze drying may be carried out at a suitable temperature depending on the freezing point of the solvent used, for example, about 12 °C when dioxane is used as a solvent.

The crystalline amlodipine camsylate used for the present invention may be produced according to the method disclosed in WO 02/079158 A1.

The present invention also provides an amorphous amlodipine camsylate composite prepared by dissolving crystalline amlodipine camsylate, surfactants, water-insoluble inorganic carriers or other additives in a suitable organic solvent and removing the solvent from the resulting solution.

Further, the present invention further provides a pharmaceutical composition for treating cardiovascular diseases comprising the inventive amorphous amlodipine camsylate as an active ingredient and pharmaceutically acceptable excipients, carriers or diluents.

The pharmaceutical composition of the present invention may be formulated in accordance with any of the conventional procedures. The formulation may be in the form of a tablet, pill, powder, sachet, elixir, suspension, emulsion, solution, syrup, aerosol, soft or hard gelatin capsule, sterile injectable solution or sterile packaged powder.

Examples of suitable carriers, excipients and diluents are starches, sucrose, lactose, dextrose, gelatin, sorbitol, mannitol, calcium silicate, cellulose, methyl cellulose, microcrystalline cellulose, polyvinylpyrrolidone, water, methylhydroxybenzoates, propylhydroxybenzoates, talc, magnesium stearate and mineral oil. The formulations may additionally include fillers, anti-agglutinating agents, disintegrants, glidants, wetting agents, flavoring agents, emulsifiers, preservatives and the like. The compositions of the invention may be formulated so as to provide quick, sustained or delayed release of the active ingredient after their administration to a mammal by employing any of the procedures well known in the art.

The pharmaceutical composition of the present invention can be administered via various routes including oral, transdermal, subcutaneous, intravenous and intramuscular introduction. In case of human, a typical daily dose of the amorphous amlodipine camsylate may range from about 1.0 to 10.0 mg/kg body weight, preferably 5.0 to 8.0 mg/kg body weight, and can be administered in a single dose or in divided doses.

However, it should be understood that the amount of the active ingredient actually administered ought to be determined in light of various relevant factors including the condition to be treated, the chosen route of administration, the age, sex and body weight of the individual patient, and the severity of the patient's symptom; and, therefore, the above dose should not be intended to limit the scope of the invention in any way.

The present invention will be described in further detail with reference to Examples. However, it should be understood that the present is not restricted by the specific Examples.

### Example

### Preparation 1: Preparation of crystalline amlodipine camsylate

12.25 g (0.03 mol) of amlodipine (Hanmi Pharm. Co. Ltd.) was dissolved in 50 mℓ of methanol, cooled to 10 °C, and a solution of 7.8 g (0.336 mol) of (1S)-(+)-10-camphor sulfonic acid (Aldrich) in 19.5 mℓ of methanol was gradually added thereto. After the reaction mixture was stirred at room temperature for 2 hours, the solid formed was filtered, washed with 25 mℓ of methanol, and dried to obtain 16.7 g (yield: 86.8%) of the title compound in the form of a white crystal.

### Preparation of amorphous amlodipine camsylate

### Example 1

7.841 mg of crystalline amlodipine camsylate obtained from Preparation 1 was dissolved in a mixed solution of ethanol/methylene chloride (20/80 (w/w)) to a concentration of about 100 mg/ml. The resulting solution was subjected to spray drying under the conditions listed below, followed by further drying at 60 °C for 1 hour and storing in a container having silica gel desiccant to obtain dry amorphous amlodipine camsylate:
< Spray Drying Conditions>
   1) Spray dryer: Buchi Minispray dryer B-191
   2) Inlet and outlet temperatures: 80 °C and 52 °C, respectively,
   3) Air flow: 500 NI/h, and
   4) Pumping rate: 12% (about 120ml spraying per an hour)

### Preparation of amorphous amlodipine camsylate composite

### Example 2

The procedure of Example 1 was repeated except that 1.159 mg of anhydrous silica was used together with 7.841 mg of crystalline amlodipine camsylate to obtain dry amorphous amlodipine camsylate composite.

### Example 3

The procedure of Example 1 was repeated except that 1.159 mg of Tween 80 was used together with 7.841 mg of crystalline amlodipine camsylate to obtain dry amorphous amlodipine camsylate composite.

### Example 4

The procedure of Example 1 was repeated except that 1.159 mg of Tween 80 and 1.000 mg of anhydrous silica were used together with 7.841 mg of crystalline amlodipine camsylate to obtain dry amorphous amlodipine camsylate composite.

### Example 5

The procedure of Example 1 was repeated except that 2.159 mg of hydroxypropylmethylcellulose was used together with 7.841 mg of crystalline amlodipine camsylate to obtain dry amorphous amlodipine camsylate composite.

### Example 6

The procedure of Example 1 was repeated except 2.159 mg of hydroxypropylmethylcellulose and 1.000 mg of anhydrous silica were used together with 7.841 mg of crystalline amlodipine camsylate to obtain dry amorphous amlodipine camsylate composite.

### Example 7

The procedure of Example 1 was repeated except that 1.159mg of Tween 80, 2.000 mg of hydroxypropylmethylcellulose and 1.000 mg of anhydrous silica were used together with 7.841 mg of crystalline amlodipine camsylate to obtain dry amorphous amlodipine camsylate composite.

### Example 8

The procedure of Example 1 was repeated except that 2.159 mg of microcrystalline cellulose was used together with 7.841 mg of crystalline amlodipine camsylate to obtain dry amorphous amlodipine camsylate composite.

### Example 9

The procedure of Example 1 was repeated except that 2.159 mg of microcrystalline cellulose, 1.000 mg of Tween 80 and 1.000 mg of anhydrous silica were used together with 7.841 mg of crystalline amlodipine camsylate to obtain dry amorphous amlodipine camsylate composite.

### Preparation of a tablet containing amorphous amlodipine camsylate

### Example 10

7.841 mg of the dry amorphous amlodipine camsylate obtained in Example 1, 119.159 mg of microcrystalline cellulose, 63mg of calcium phosphate, 6 mg of sodium starch glycolate and 2 mg of magnesium stearate were mixed and the resulting mixture was then pressed into tablets using a conventional tablet making machinery.

### Example 11

The procedure of Example 10 was repeated except that 40 mg of Tween 80 was further used.

### Experimental Example 1: X-ray Diffraction and Differential Scanning Calorimetry (DSC) Analysis

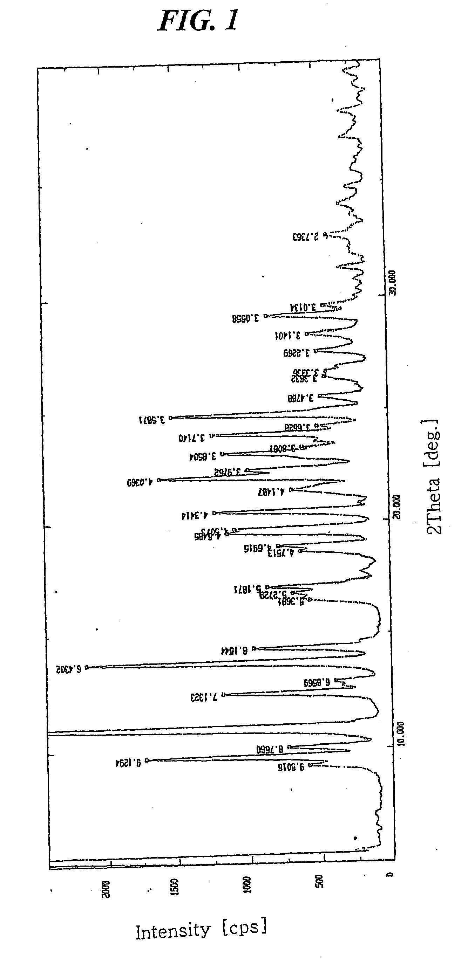

Crystalline amlodipine camsylate obtained in Preparation 1 and the inventive amorphous amlodipine camsylate obtained in Example 1 were analyzed with an X-ray diffractometer and DSC. The results are shown in FIGS. 1 to 4.

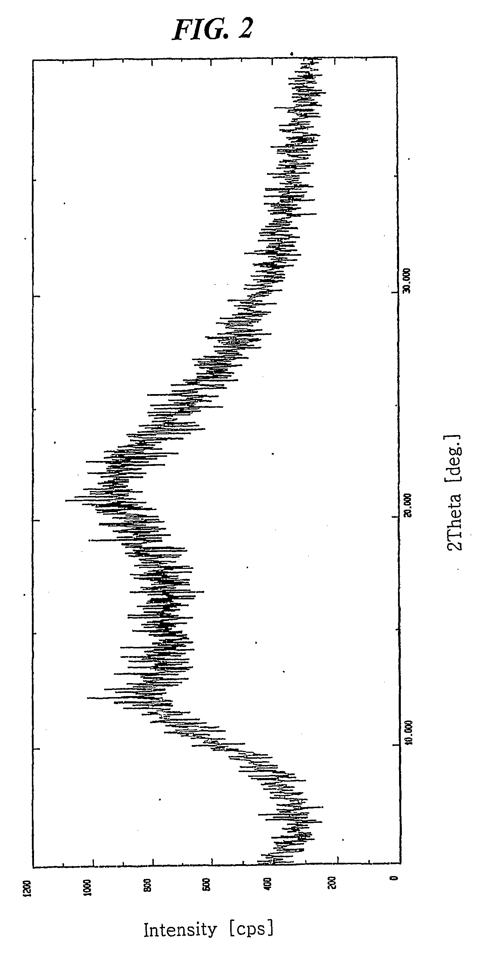

From FIGS. 1 and 2 showing X-ray diffraction scans of the crystalline amlodipine camsylate and the inventive amlodipine camsylate, it can be seen that the inventive amlodipine camsylate is amorphous.

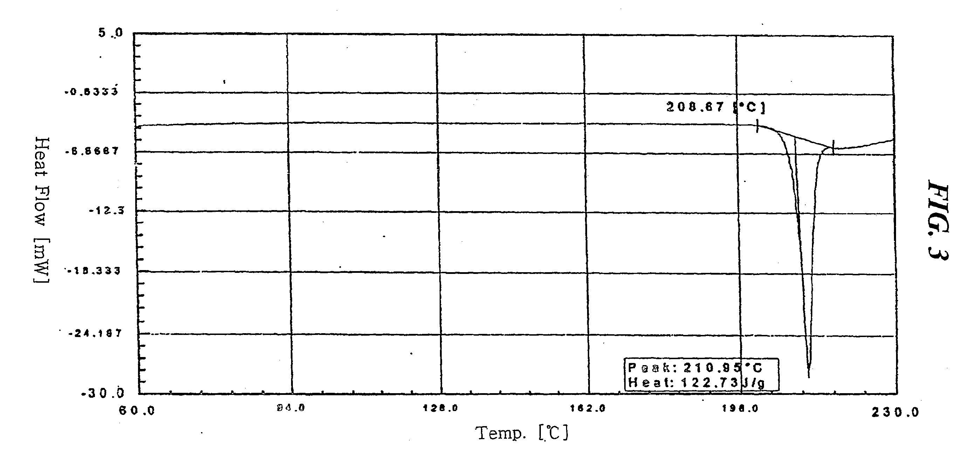

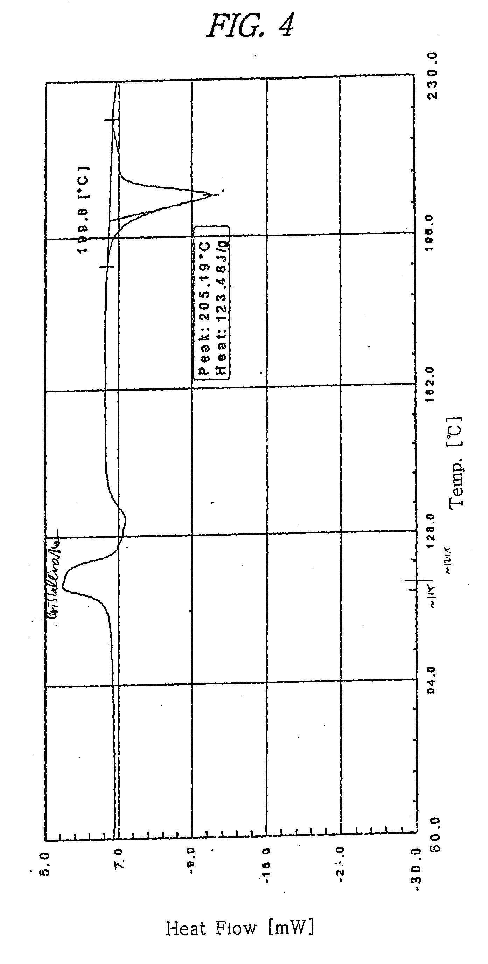

Generally, an amorphous material has absorption peaks at low temperature due to unstableness but as can be shown in Figs 3 and 4, the absorption peak temperature of the inventive amorphous amlodipine camsylate is similar to that of the crystalline amlodipine camsylate. That is, the inventive amorphous amlodipine camsylate has a sufficient thermodynamic stability.

### Experimental Example 2: Solubility Test

An amlodipine salt preferably has a solubility in water of more than 1 mg/mℓ at pH 1 to 7.5, particularly at the blood pH value of 7.4. Accordingly, the solubility and saturation pH of the inventive amorphous amlodipine camsylates prepared in Example 1 were measured and compared with those of amlodipine besylate (Korean Patent Publication No. 1995-7228) and crystalline amlodipine camsylate obtained in Preparation 1 (WO 02/079158 A1). The measurement was performed according to the procedure described in the Korean Pharmacopoeia (Korean Ministry of Health and Welfare, General principle of medical supplies, Vol. 1, Clause 29, the 7th revision) which comprises the steps of dissolving each compound in distilled water to saturation, analyzing the saturated solution with liquid chromatography, and measuring the dissolved amount of each compound based on the amount of amlodipine. The results are shown in Table 1.

**Table 1**

| Salt | Solubility (mg/mℓ) | Saturation pH |
|---|---|---|
| Crystalline amlodipine besylate | 2.35 | 6.2 |
| Crystalline amlodipine camsylate | 1.42 | 6.2 |
| Amorphous amlodipine camsylate | 6.59 | 6.2 |

As shown in Table 1, the solubility of the inventive amorphous amlodipine camsylate is 3 to 5 times higher than that of crystalline amlodipine besylate and crystalline amlodipine camsylate at the same saturation pH.

### Experimental Example 3: Stability Test

A tablet or capsule ought to be stable in the solid state. Accordingly, the time-dependent stability of the tablet obtained in Example 10 was measured and compared with that of an amorphous amlodipine besylate tablet. Specifically, each tablet was stored under accelerated aging conditions (a temperature of 40± 2 °C and a relative humidity of 75± 5 %), and after 2 and 4 months, the remaining amount of active amlodipine was determined with a liquid chromatography. The results are shown in Table 2.

**Table 2**

| Test tablet | Initial | 2 months | 4 months |
|---|---|---|---|
| Amorphous amlodipine camsylate tablet | 100.3± 0.9% | 99.0± 0.7% | 98.0± 1.3% |
| Amorphous amlodipine besylate tablet | 102.7± 1.3% | 92.7± 1.8% | - |

As shown in Table 2, amorphous amlodipine besylate tablet underwent about 10% degradation during 2 months, while the inventive amorphous amlodipine camsylate tablet was stable.

While the invention has been described with respect to the specific embodiments, it should be recognized that various modifications and changes may be made by those skilled in the art to the invention which also fall within the scope of the invention as defined as the appended claims.

## Claims

1. An amorphous amlodipine camsylate prepared by dissolving a crystalline amlodipine camsylate in an organic solvent selected from the group consisting of acetone, methanol, ethanol, acetonitrile, tetrahydrofurane, dioxane, methyl acetate, ethyl acetate, methylene chloride, chloroform and a mixture thereof, and removing the solvent from the resulting solution.

2. A pharmaceutical composition for treating a cardiovascular disease comprising the amorphous amlodipine camsylate of claim 1 as an active ingredient and a pharmaceutically acceptable carrier,

## Patentansprüche

1. Amorphes Amlodipincamsylat, hergestellt durch Auflösen eines kristallinen Amlodipincamsylats in einem organischen Lösungsmittel, das ausgewählt ist aus der Gruppe bestehend aus Aceton, Methanol, Ethanol, Acetonitril, Tetrahydrofuran, Dioxan, Methylacetat, Ethylacetat, Methylenchlorid, Chloroform und einer Mischung derselben, und Entfernen des Lösungsmittels von der resultierenden Lösung.

2. Pharmazeutische Zusammensetzung zur Behandlung einer kardiovaskulären Erkrankung umfassend das amorphe Amlodipincamsylat nach Anspruch 1 als einen aktiven Bestandteil und einen pharmazeutisch annehmbaren Träger.

## Revendications

1. Un camsylate d'amlodipine amorphe préparé en dissolvant un camsylate d'amlodipine cristallin dans un solvant organique sélectionné parmi le groupe constitué par l'acétone, le méthanol, l'éthanol, l'acétonitrile, le tétrahydrofurane, le dioxane, l'acétate de méthyle, l'acétate d'éthyle, le chlorure de méthylène, le chloroforme et un mélange de ces derniers, et en extrayant le solvant de la solution résultante.

2. Une composition pharmaceutique pour le traitement d'une maladie cardiovasculaire comprenant le camsylate d'amlodipine amorphe selon la revendication 1 en tant qu'ingrédient actif et un excipient pharmaceutiquement acceptable.
